# EUROPEAN PATENT APPLICATION

(11) **EP 3 488 858 A1**
(43) Date of publication of application: **29.05.2019**
(21) Application number: 17306642.4
(22) Date of filing: 27.11.2017
(51) Int. Cl.: A61K 38/36, A61K 38/37, A61P 35/00, A61P 39/00, A61P 1/00, A61P 27/02

(54) **A VON WILLEBRAND FACTOR COMPOSITION FOR USE IN TREATING A PATHOLOGY MEDIATED BY ANGIOGENESIS**

(71) Applicant: Laboratoire Français du Fractionnement et des Biotechnologies, 91940 Les Ulis (FR)
(72) Inventor: RANDI, Anna Maria, London, NW6 3PH (GB); STARKE, Richard, London, SW19 2EA (GB); SMITH, Koval, London, W12 9LU (GB)
(74) Representative: IPAZ

(57) **Abstract**

The present invention relates to a von Willebrand factor composition for the prophylaxis and/or the treatment of a pathology mediated by angiogenesis.

## Description

The present invention relates to a novel use of a von Willebrand factor composition comprising a low content of coagulation factor VIII, and in particular for its use in preventing and/or treating patient with a pathology mediated by angiogenesis.

Von Willebrand factor (vWF) has been known for many decades as a critical regulator of the haemostatic system, linked to both bleeding and thrombotic disorders. In recent years, new functions of von Willebrand factor have emerged, which are transforming our understanding of its role in the vasculature and have opened new avenues for therapeutic intervention.

In some patients with decreased or dysfunctional vWF (in case of congenital or acquired von Willebrand disease or vWD), vascular malformations with fragile, disrupted vessels, linked to abnormal angiogenesis, have been described. These lesions, most commonly observed in the gastrointestinal (GI) tract (angiodysplasia), can cause severe, intractable bleeding in patients with vWD. von Willebrad disease is classically treats by administration to the patient of an effective dose of Haemate P® (CSL Limited). Haemate P® is a composition of von Willebrand factor and factor VIII, which comprises 160 UI/mL of von Willebrand factor and 66,6UI/mL of factor VIII. The treatment with Haemate P® made it possible to treat gastro-intestinal haemorrhage and/or bleeding, but requires very close monitoring of thromboses, due to the presence of factor VIII in the therapeutic concentrate. As a result, therefore, although it was possible to treat haemorrhages in the patient being studied with an administration of the composition of Haemate P®, the administration of this product can lead to the occurrence of thrombosis.

Consequently, it would appear necessary to provide compounds useful in a method for the prophylaxis and/or a method for the treatment of a pathology mediated by angiogenesis, without the fear of causing an additional thrombosis and without the fear of a possible relapse, i.e. without the fear of developing new haemorrhages or new bleeding.

As used herein, the term "angiogenesis" means the generation of new blood vessels into a tissue or organ. Under normal physiological conditions, humans or animals only undergo angiogenesis in very specific restricted situations. For example, angiogenesis is normally observed in wound healing, foetal and embryonal development and formation of the corpus luteum, endometrium and placenta. The control of angiogenesis is a highly regulated system of angiogenic stimulators and inhibitors. The control of angiogenesis has been found to be altered in certain disease states and, in many cases, the pathological damage associated with the disease is related to the uncontrolled angiogenesis.

Both controlled and uncontrolled angiogenesis are thought to proceed in a similar manner. Endothelial cells and pericytes, surrounded by a basement membrane, form capillary blood vessels. Angiogenesis begins with the erosion of the basement membrane by enzymes released by endothelial cells and leukocytes. The endothelial cells, which line the lumen of blood vessels, then protrude through the basement membrane. Angiogenic stimulants induce the endothelial cells to migrate through the eroded basement membrane. The migrating cells form a "sprout off" the parent blood vessel, where the endothelial cells undergo mitosis and proliferate. The endothelial sprouts merge with each other to form capillary loops, creating the new blood vessel. In the disease state, prevention of angiogenesis could avert the damage caused by the invasion of the new microvascular system.

Persistent, unregulated angiogenesis occurs in a multiplicity of disease states, tumor metastasis and abnormal growth by endothelial cells and supports the pathological damage seen in these conditions.

The inventors have shown, surprisingly, that the administration of a pharmaceutical composition comprising von Willebrand factor and a low content of factor VIII could potentially prevent patient from a pathology mediated by angiogenesis or to treat patient with a pathology mediated by angiogenesis, without the fear of causing an additional thrombosis and without the fear of developing new haemorrhages or new bleeding. More particularly, the inventors have shown that the composition of the invention comprising von Willebrand factor and a low content of factor VIII will be able to regulate angiogenesis by stabilizing β3 integrin and modulating Ang-2 level in endothelial cells.

Furthermore, the inventors have shown, surprisingly, that the administration of a pharmaceutical composition comprising von Willebrand factor and a low content of factor VIII would inhibit the angiogenesis in endothelial cells that are deficient in von Willebrand factor but not in healthy endothelial cells. On the contrary, other plasmatic von Willebrand factor concentrates may inhibit angiogenesis in every endothelial cells, deficient in von Willebrand factor or not. In consequence, the use of a von Willebrand factor composition comprising a low content of factor VIII in an antiangiogenic treatment would result in the absence or lower of side effects.

The invention therefore relates to a pharmaceutical composition comprising von Willebrand factor for use in the prophylaxis and/or the treatment of a pathology mediated by angiogenesis, wherein the content of factor VIII in the pharmaceutical composition is lower than or equal to 30 IU/ml.

Within the meaning of the present invention, by "pharmaceutical composition" is meant a pharmaceutical composition in liquid form, in particular in the form of a solution or a suspension as well as a pharmaceutical composition in dried powder form or in lyophilized form. This pharmaceutical composition comprises at least von Willebrand factor, factor VIII and pharmaceutically acceptable excipients.

The term "von Willebrand factor" or "vWF" includes the polypeptides comprising the sequence of wild-type human von Willebrand factor or of von Willebrand factor derived from another species (for example bovine, porcine, canine or murine). It also comprises the natural allelic variations of von Willebrand factor which can exist and any form or degree of glycosylation or other post-translational modification. The term "von Willebrand factor" also includes the variants of von Willebrand factor which have the same or a higher biological activity with respect to the activity of the wild form, these variants in particular have at least 70%, preferably 75%, preferably 80%, preferably 85%, preferably 90%, preferably 92%, preferably 94%, preferably 95%, preferably 96%, preferably 97%, preferably 98%, preferably 99%, preferably 100% homology with the nucleotide sequence of wild-type von Willebrand factor. Advantageously, the von Willebrand factor is a human von Willebrand factor.

In an advantageous embodiment, the amount of factor VIII in the pharmaceutical composition comprising von Willebrand factor according to the invention is less than or equal to 20 IU/ml, preferably less than or equal to 10 IU/ml. This level is measured using the von Willebrand ristocetin cofactor assay method (vWF:RCo) with respect to the international standard of concentrated von Willebrand factor defined by the World Health Organisation (WHO). The activity of the von Willebrand factor in the composition of the invention is preferably 100 IU for 1 ml of reconstituted solution. The content of factor VIII in the composition according to the invention is therefore divided by a minimum of a factor of 4 with respect to the products on the market, thus making it possible to limit the risk of thrombosis by controlling and reducing as far as possible the exogenous supply of FVIII. The amount of factor VIII in the pharmaceutical composition comprising von Willebrand factor according to the invention is preferably less than or equal to 10 IU/ml, preferably less than 8 IU/ml, preferably less than 6 IU/ml, preferably less than 4 IU/ml. In a particular embodiment, the composition comprising von Willebrand factor is entirely free from blood clotting factor VIII.

In another embodiment, the factor VIII / von Willebrand factor ratio is advantageously between 1/10 and 5/10 IU/ml.

In an advantageous embodiment, the pharmaceutical composition comprising von Willebrand factor intended for use in the prophylaxis and/or the treatment of a pathology mediated by angiogenesis is free of ADAMTS13. The term "free of ADAMTS13" or "free of ADAMTS13 protein" means that the composition of the invention does not contain any ADAMTS13 protein or that this ADAMTS13 protein is present in a negligible quantity or that the ADAMTS13 activity is negligible. In fact, the inventors found that this protein has a tendency to weaken the von Willebrand factor, and even to degrade it, and also that the activity of this protein is increased by the shearing forces induced by the mechanical circulatory support device. Thus, the absence of this protein or its presence in a negligible quantity made it possible to reduce the risks of the occurrence of haemorrhage and/or bleeding in patients with a pathology mediated by angiogenesis.

Advantageously, the pharmaceutical composition comprising von Willebrand factor is free of ADAMTS13.

In another particular embodiment, the residual amount of ADAMTS13 in the pharmaceutical composition comprising von Willebrand factor according to the invention is less than or equal to 0.15 µg/ml. Preferably, the residual amount of ADAMTS13 is less than or equal to 0.10 µg/ml, preferably less than or equal to 0.05 µg/ml, preferably less than or equal to 0.01 µg/ml. The residual amount of ADAMTS13 is preferably between 0.01 µg/ml and 0.15 µg/ml. Advantageously, the pharmaceutical composition comprising von Willebrand factor does not contain ADAMTS13 activity.

In another particular embodiment, the residual level of ADAMTS13 activity in the pharmaceutical composition comprising von Willebrand factor according to the invention is less than or equal to the detection limit, i.e. less than or equal to 0.03 ADAMTS13:Act [U/ml].

This level is measured using the method described in Kokame et al. (British Journal of Haematology, 2005, vol. 129, pp. 93-100), using FRET (fluorescence resonance energy transfer) and the fluorogenic substrate FRETS-vWF73 (Peptides International, Louisville, USA). The amount of residual ADAMTS13 in the composition according to the invention is at least 2 to 3 times lower with respect to the products on the market which comprise at least 0.13 ± 0.07 ADAMTS13: Act [U/ml], thus making it possible to avoid any risk of the occurrence of haemorrhage and/or bleeding in patients with a pathology mediated by angiogenesis. In a preferred embodiment, the residual amount of ADAMTS13 in the pharmaceutical composition comprising von Willebrand factor according to the invention is less than 0.10 ADAMTS13:Act [U/ml], preferably less than 0.05 ADAMTS13:Act [U/ml]. In a particular embodiment, the composition comprising von Willebrand factor is entirely free of ADAMTS13. In another embodiment of the invention, the quantity of ADAMTS13 in the composition comprising von Willebrand factor of the invention is adjusted in order to avoid the presence of ultra large multimers (>20 mers). In this case, the ADAMTS13 can advantageously be of plasmatic origin and copurified with the composition comprising von Willebrand factor of the invention, or be of recombinant origin and advantageously be added to the composition comprising von Willebrand factor of the invention of recombinant origin.

The distribution of the multimeric forms of von Willebrand factor is defined after analysis of an electrophoresis gel making it possible to quantify the size of the mers (sub-units) in multiples of the monomer sub-unit of 225 kD. Mers with a size of 225 x 2 to 225 x 15 are thus described. By high-molecular weight multimers of the pharmaceutical composition comprising von Willebrand factor is meant multimers starting from 10 monomers.

In a particular embodiment, the content of high-molecular weight multimers in the pharmaceutical composition comprising von Willebrand factor is close to that of plasma.

In another particular embodiment, the content of high-molecular weight multimers in the pharmaceutical composition comprising von Willebrand factor is sufficiently maintained in order to allow sufficient *in vivo* activity of the pharmaceutical composition.

Advantageously, the pharmaceutical composition comprising von Willebrand factor according to the invention has a content of high-molecular weight multimers of at least 65%, preferably 70%, yet more preferably 75%, particularly preferably 80% of the total multimer content of von Willebrand factor contained in the pharmaceutical composition. The presence of at least 65% of high-molecular weight multimers confers to the composition according to the invention a better therapeutic efficacy.

The pharmaceutical composition according to the invention can comprise in addition one or more excipients, making it possible to stabilize the von Willebrand factor and making it possible to solubilize a lyophilized form of the von Willebrand factor.

In particular, the excipients can be chosen from:
- a hydrophilic amino acid or one bearing a positively-charged side chain,
- optionally a hydrophobic amino acid,
- a salt,
- a protein stabilizer, or
- a mixture thereof.

The hydrophilic (or polar) amino acids or the amino acids bearing a positively-charged side chain include lysine, arginine, histidine, glycine, serine, threonine, tyrosine, asparagine and glutamine. Among the hydrophilic amino acids or those bearing a positively-charged side chain, arginine is preferably used, or one of the salts derived therefrom such as arginine hydrochloride or also arginine phosphate. The amino acids such as glycine and/or lysine, or one of the salts derived therefrom such as lysine hydrochloride can advantageously be added. Examples of the hydrophobic amino acids include the following amino acids: alanine, valine, leucine, isoleucine, phenylalanine, tryptophan and proline.

The salt can be an alkali metal salt, an alkaline earth metal salt or a transition metal salt. In particular, sodium citrate, calcium chloride and zinc chloride can be mentioned as example of salt. In a preferred embodiment, the salt used is preferably sodium citrate or calcium chloride.

The protein stabilizer can be chosen from among the known protein stabilizers, for example albumin and factor VIII. Advantageously, the preferred protein stabilizer is albumin, preferably human albumin.

Advantageously, the composition comprises von Willebrand factor as active ingredient and the following pharmaceutically acceptable excipients: albumin, arginine hydrochloride, glycine, sodium citrate and calcium chloride.

More particularly, the composition can comprise:
- human von Willebrand factor;
- albumin, preferably human albumin;
- arginine, optionally in the form of the hydrochloride;
- glycine;
- sodium citrate;
- calcium chloride.

Reconstitution from powder in the form of an injectable preparation can be carried out by adding water for injections (WFI water).

According to the present invention, the term "prophylaxis" or "prevention" or "preventative treatment" or "prophylactic treatment" comprises a treatment leading to the prevention of a disease as well as a treatment reducing and/or delaying the incidence of a disease or the risk of it occurring. According to the invention, the von Willebrand factor composition is particularly useful for preventing a pathology mediated by angiogenesis.

The term "treatment" or "curative treatment" is defined as a treatment leading to a cure or a treatment which alleviates, improves and/or eliminates, reduces and/or stabilizes the symptoms of a disease or the suffering that it causes. According to the invention, the von Willebrand factor composition is particularly useful for treating a pathology mediated by angiogenesis.

In a preferred embodiment, the pathology mediated by angiogenesis according to the present invention can be a cancer pathology. Advantageously, the cancer pathology mediated by angiogenesis can be a solid tumour, a blood-borne tumour, a benign tumour or a tumour metastasis. Advantageously, the cancer pathology mediated by angiogenesis can be breast cancer, leukaemia, Kaposi's sarcoma, haemangioma, rhabdomyosarcoma, retinoblastoma, Ewing's sarcoma, neuroblastoma, osteosarcoma, acoustic neuroma, neurofibroma, trachoma, pyogenic granulomas or other neoplastic diseases of the bone marrow.

In another preferred embodiment, the pathology mediated by angiogenesis according to the present invention can be a macular degeneration, macular degeneration of age, trachoma, diabetic retinopathy, neovascular glaucoma, retrolental fibroplasia of, proliferative vitreoretinopathy, psoriasis, Kaposi's syndrome, ulcerative colitis, retinopathy of prematurity, of epidemic keratoconjunctivitis, atopic keratitis, the superior limbic keratitis, dry keratoconjunctivitis (pterygium keratitis sicca) of vitamin A deficiency, corneal graft rejection, of A condition resulting from excessive contact lens wear, Sjögrens syndrome, acne rosacea, couperose, phlyctenular keratitis, syphilis, lipid degeneration, infections with Herpes simplex virus, infections by Herpes zoster viruses, protozoal infections, infections of mycobacteria, infections causing a retinitis or choroiditis, chemical burns, bacterial ulcers, fungal ulcers, Mooren's ulcer, of the Terrien's marginal degeneration, marginal keratolysis of from polyarteritis, scleritis, Stevens-Johnson of the disease, radial keratotomy, trauma, systemic lupus, Wegener sarcoidosis, sarcoidosis, of sickle erythrocytes, the vein occlusion, artery occlusion, sickle cell anemia, pseudoxanthoma elasticum, pemphigoid disease, Paget's disease, obstruction of the carotid artery disease, the Chronic vitreous disease, Lyme disease, Eales's disease, Behcet's disease, presumed ocular histoplasmosis, Best's disease, myopia, optic bites, Stargardt disease, disease pars planitis, chronic retinal detachment, optic pits, hyperviscosity syndromes, the taxoplasmose, post-laser complications, abnormal proliferation of fibrovascular or fibrous tissue, bartonellosis, Osler-Weber-Rendu disease, acquired immunodeficiency syndrome, ocular neovascular disease, osteoarthritis, diseases from chronic inflammation, Crohn's disease, atherosclerosis, pyrogenic granulomas and rubella.

In another preferred embodiment, the pathology mediated by angiogenesis according to the present invention is not von Willebrand disease.

In a preferred embodiment, the pharmaceutical composition comprising von Willebrand factor is administered to the patient with a pathology mediated by angiogenesis at an efficient dose.

By "administration" is meant the injection to the patient of the pharmaceutical composition according to the invention. This administration comprises parenteral injections, such as in particular intravenous, intramuscular, subcutaneous, intraorbital, intradermal, intra-spinal and intraperitoneal injections as well as infusion directly into a tissue or an organ. Administration orally or by using the airways, for example by inhalation, is also envisaged within the scope of the present invention. Particularly advantageously, the pharmaceutical composition comprising von Willebrand factor is administered intravenously.

In a particularly advantageous embodiment, the present invention relates to the use of a pharmaceutical composition comprising von Willebrand factor in the manufacture of a medicinal product for preventing or treating patient with a pathology mediated by angiogenesis.

Advantageously, the method for the curative treatment of a pathology mediated by angiogenesis comprises the administration to said patient of a von Willebrand factor composition according to the invention.

The von Willebrand factor composition can be obtained by processes well known to a person skilled in the art, for example by fractionation of plasma, by expression in cell culture or by expression in the milk of transgenic animals.

According to a particular embodiment, the von Willebrand factor of the invention can be obtained from human blood plasma, either from a fraction of cryoprecipitated human plasma or from the supernatant of cryoprecipitated plasma again containing von Willebrand factor or obtained by conventional fractionation methods (Cohn et al., J. Am. Chem. Soc., 68, 459, 1946 and Kistler et al., Vox Sang., 7, 1962, 414-424), possibly having been subjected to a pre-purification treatment in particular by adsorption on aluminium hydroxide, in which the vWF is complexed to factor VIII. This is then called "plasmatic" von Willebrand factor.

In a particular embodiment, the von Willebrand factor is obtained from a fraction of cryoprecipitated plasma that has been subjected to a prior purification step by chromatography using an anion exchanger of the DEAE-Fractogel®-TSK 650 type, as described in the patents EP 0 359 593 and EP 0 503 991.

According to another particular embodiment, the von Willebrand factor can be obtained by genetic engineering, in particular produced by cells the DNA of which has been modified by genetic recombination in such a way as to express a molecule of vWF, and having the particular characteristics of glycosylation. This is then called recombinant von Willebrand factor. Thus, the von Willebrand factor of the invention is derived from the transcription then the translation of a molecule of DNA coding for the von Willebrand factor in a host cell. The recombinant von Willebrand factor of the invention can be obtained using standard techniques well known to a person skilled in the art, allowing the expression of a protein in a biological system. More particularly, by "recombinant von Willebrand factor" is meant any von Willebrand factor obtained by genetic recombination and expressed by a cultured cell line. The following lines can be mentioned by way of example: BHK (Baby Hamster Kidney) and in particular BHK tk"tsl3 (CRL 10314, Waechter and Baserga, Proc. Natl. Acad. Sci. USA 79:1106-1110, 1982), CHO (ATCC CCL 61), COS-I (ATCC CRL 1650), HEK293 (ATCC CRL 1573; Graham et al, J. Gen. Virol. 36:59-72, 1977), Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1) and DUKX cells (CHO cell line) (Urlaub and Chasin, Proc. Natl. Acad. Sci. USA 77:4216-4220, 1980), 3T3 cells, Namalwa cells, or BHK cells that have been adapted to grow in the absence of serum (document US 6,903,069). Recombinant von Willebrand factor can thus be isolated from a fraction enriched with vWF and/or with factor VIII/recombinant vWF complex isolated from the supernatant of cell cultures according to known techniques.

In another particular embodiment of the invention, the von Willebrand factor is a transgenic von Willebrand factor, i.e. obtained by genetic recombination and expressed by a living tissue, either animal or plant.

Advantageously, the transgenic von Willebrand factor is obtained by genetic recombination and expressed in an animal, called a transgenic animal. By transgenic animal is meant an animal into the genome of which one or more genes has been introduced by transgenesis. Preferably, the von Willebrand factor is in particular produced in the milk of a transgenic animal, the formulation of the invention making it possible to retain a satisfactory level of biological activity of the von Willebrand factor after the lyophilization thereof. According to a preferred embodiment, the human von Willebrand factor is produced in the milk of non-human female transgenic mammals, genetically modified in order to produce this protein. The rabbit, goat, cow, rodent, hamster, camel, llama, dromedary camel, mouse, rat, pig, sow, horse, dog, cat, ewe, ruminants, hog etc., the list not being limitative, can in particular be mentioned as examples of genetically modified non-human transgenic mammals capable of producing this protein. It is preferably the milk of a transgenic rabbit or goat. The secretion of von Willebrand factor by the mammary glands, making possible the secretion thereof in the milk of the transgenic mammal, implies that the expression of the von Willebrand factor can be controlled in a tissue-dependent manner. Such control methods are well known to a person skilled in the art. The expression is controlled by the sequences allowing the expression of the protein in a particular tissue of the animal. These are, in particular, the WAP promoter sequences, beta casein sequences, beta lactoglobulin sequences and signal peptide sequences. The process for extracting the proteins of interest from the milk of the transgenic animals is described in the patent EP 0 264 166. Transgenic von Willebrand factor can also be isolated from a fraction enriched with vWF and/or with factor VIII/vWF complex obtained from the milk of transgenic mammals as described in the patent US 6 518 482.

Whatever the method of obtaining the von Willebrand factor, from plasma or by genetic engineering (recombinant or transgenic), the von Willebrand factor fraction can be diafiltered in order to incorporate the appropriate excipients intended to allow the von Willebrand factor to be heated to dryness without the risk of denaturation, concentrated by ultrafiltration, packaged in flasks and lyophilized, after a prior addition of an additional pharmaceutically acceptable stabilizer, such as albumin.

Finally, the lyophilizates undergo a final step of viral inactivation by heating the lyophilizate to dryness according to standard conditions, at 80°C for 72 hours, in order to inactivate the non-enveloped viruses which have not been inactivated and/or eliminated by at least one of the two preceding steps for the inactivation and/or elimination of viruses. The lyophilizates heated to dryness are then reconstituted in an aqueous medium compatible with clinical use, preferably in 10 ml of water for injection (WFI) in order to obtain an injectable formulation.

### FIGURES

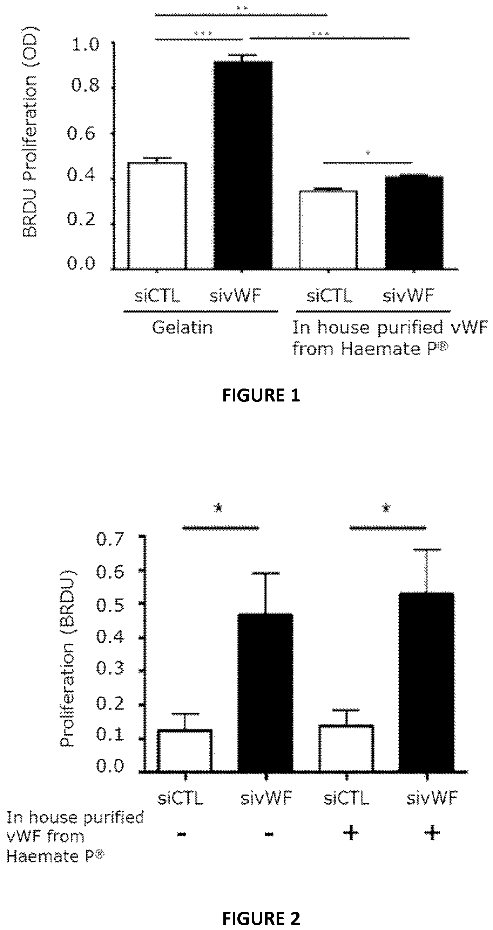

**Figure 1****:** In house purified vWF, from Haemate P®, inhibits endothelial proliferation of control and vWF-deficient endothelial cells (EC). vWF-specific (20nM; sivWF, closed bars), or control siRNA (20nM; siCTL, open bars) was transfected into HUVECs for 24 hours. Cells were then reseeded at low density on gelatin (1%) or in house purified vWF, from Haemate P® (30µg/ml) for 72 hours. Proliferation was measured by incorporation of bromodeoxyuridine (BRDU). The increase in proliferation seen in vWF deficient EC on gelatin was reduced by 1.66 fold when cells were seeded on plasma-derived vWF. Data normalized to siCTL treated cells on gelatin. Error bars = mean ±SEM. *P < 0.05; **P <0 .01, ***P<0.001 (Student t test).
**Figure 2****:** In house purified vWF, from Haemate P®, added in solution does not affect endothelial proliferation. Control or vWF deficient EC were seeded on gelatin in the presence and absence of in house purified vWF, from Haemate P®, (10µg/ml). Proliferation was measured with bromodeoxyuridine (BRDU).

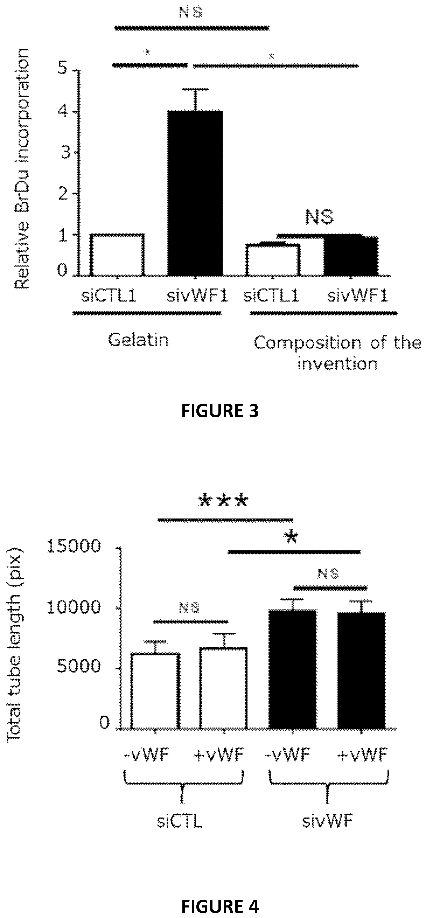

**Figure 3****:** Pharmaceutical composition of the invention normalizes proliferation of vWF-deficient EC (n=3). Data normalized to siCTL1 treated cells on gelatin (1%). The increase in proliferation seen in vWF deficient EC on gelatin was reduced by 3.12 fold when cells were seeded on the pharmaceutical composition of the invention (30µg/ml). Error bars = mean ± SEM. *P < 0.05; **P <0.01; (Student t test).
**Figure 4****:** The composition of the invention does not affect capillary network formation on Matrigel. vWF-specific (20nM; sivWF, closed bars), or control siRNA (20nM; siCTL, open bars) was transfected into HUVECs for 24 hours then seeded onto Matrigel. Capillary network formation was observed in the presence or absence of 10 µg/mL the composition of the invention after 24 hours and quantified by measuring total tube length in micrometers. (Bar = 200 µm). (n = 3). Error bars = mean ± SEM. *P <0.05; **P < 0.01; ***P < 0.001 (Student t test).

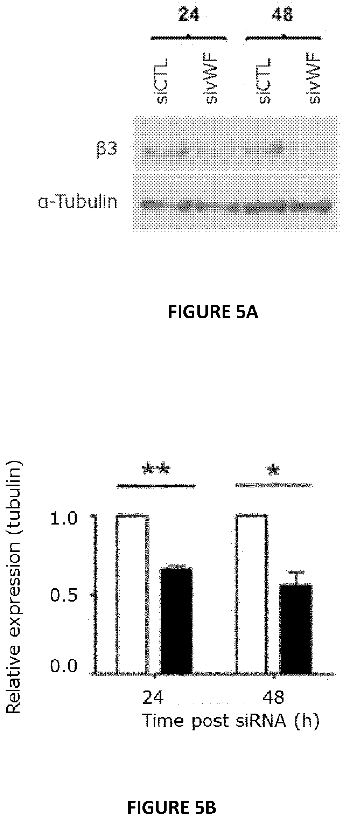

**Figure 5A****:** vWF-deficient cells display reduced β3 integrin expression and αvβ3-dependent adhesion. β3 integrin protein expression was measured by Western blotting. Figure 5A has been published in the publication of Starcke et al., Blood, January 2011, vol 117, n°3, pages 1071 - 1080, see in particular page 1075, Figure 3.
**Figure 5B****:** vWF-deficient cells display reduced β3 integrin expression and αvβ3-dependent adhesion. β3 integrin protein expression was quantified by densitometry relative to tubulin. Figure 5B has been published in the publication of Starcke et al., Blood, January 2011, vol 117, n°3, pages 1071 - 1080, see in particular page 1075, Figure 3B.

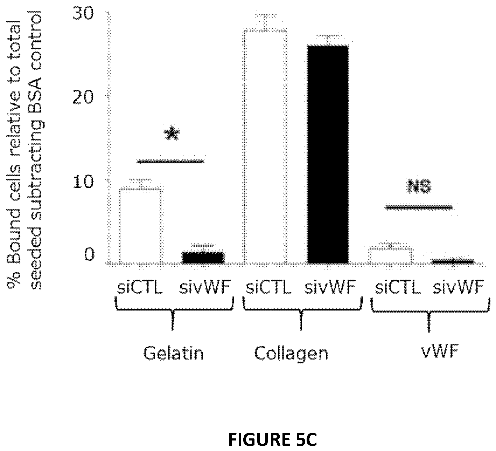

**Figure 5C****:** Control or sivWF-treated cells, 48 hours after transfection, were seeded onto different extracellular matrix substrates. After 40 minutes, nonadherent cells were removed by gentle washing and the number of bound cells was quantified relative to the total number seeded. Figure 5C has been published in the publication of Starcke et al., Blood, January 2011, vol 117, n°3, pages 1071 - 1080, see in particular page 1075, Figure 3D.

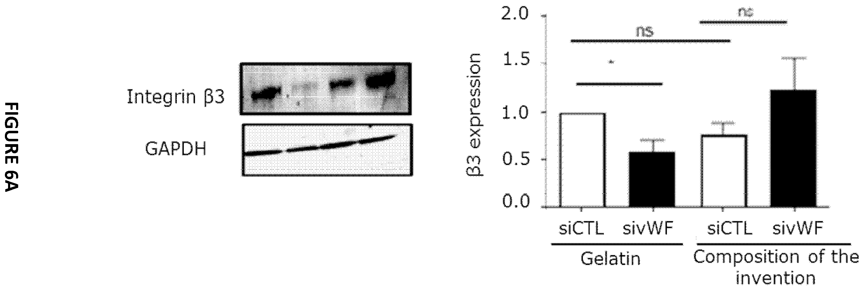

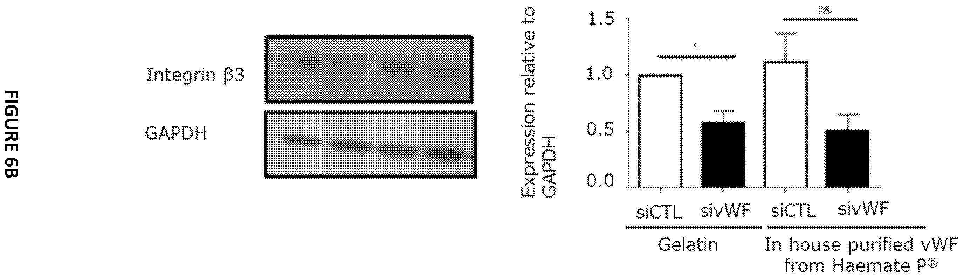

**Figure 6****:** Reduced β3 integrin expression in vWF deficient cells is normalised by the composition of the invention (A) and by in house purified vWF, from Haemate P® (B). vWF deficient EC were seeded on gelatin or vWF (1µg/ml) for 24 hours and processed for western blotting. Seeding vWF deficient EC on VWF increased the expression of β3 integrin back to basal levels. (n=4)

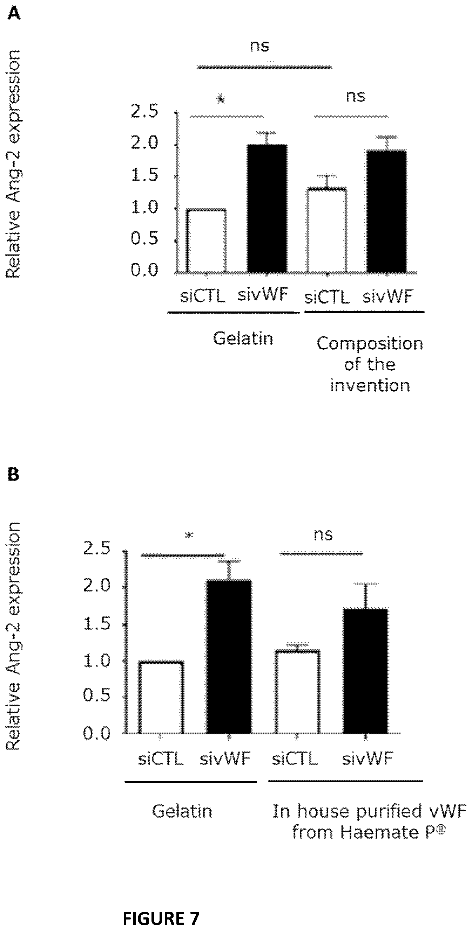

**Figure 7****:** vWF suppresses Ang62 expression in HUVEC. HUVEC treated with vWF siRNA or control for 24 hours where reseeded onto the composition of the invention (A) or in house purified vWF, from Haemate P® (B) for 24 hours. RNA was collected for analysis of Ang-2 mRNA shows that both vWF preparations can partly rescue the levels of Ang-2 in vWF-deficient cells. (n=4)

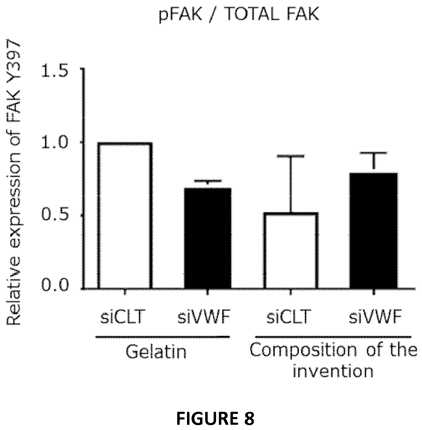

**Figure 8****:** The composition of the invention appears to normalise FAK phoshphorylation in vWF-deficient cells. vWF deficient EC were seeded on gelatin or the composition of the invention (1µg/ml) for 24 hours and processed for western blotting. (n=2)

This invention is further illustrated by the following examples, which are not to be construed in any way as imposing limitations upon the scope thereof.

### EXAMPLES

### Example 1: Preparation of a von Willebrand factor composition according to the invention

### 1) Obtaining a fraction containing vWF

A cryoprecipitate of human plasma is used, resuspended in an aqueous heparin sodium solution (2 U/ml), at a pH of 7-7.1.

This cryoprecipitate solution is subjected to a pre-purification on aluminium hydroxide in order to eliminate the main contaminants, as described in the patent EP 0 359 593. The pre-purified supernatant is then recovered and is subjected to a standard viral inactivation treatment by solvent-detergent, in the presence of Tween®-TNBP.

The pre-purified cryoprecipitate solution is then injected into a Fractogel® TSK-DEAE 650 (M) type chromatography column with a length of 25 cm and a diameter of 1 cm, equilibrated beforehand with a buffer constituted by 0.01 M trisodium citrate, 0.001 M calcium chloride, 0.11 M sodium chloride, 0.12 M glycine and 0.016 M lysine, adjusted to a pH of 7.01, the linear velocity of the mobile phase of which is set at 100 cm/hour. The von Willebrand factor, the factor VIII and the fibronectin are retained on the chromatographic support. The weakly-retained proteins or those not retained by the support, principally the fibrinogen and the immunoglobulin Gs (IgGs), are eliminated in the filtrate, as well as the Tween® and TNBP, by several successive washings with the same buffer.

The protein content is monitored by measuring the absorption at 280 nm (labelled O.D. below).

When the O.D., measured at 280 nm, has returned to the baseline, the concentration of sodium chloride in the buffer is increased to 0.15 M. Under these conditions, the von Willebrand factor is eluted. The eluate thus obtained is very rich in von Willebrand factor and in fibronectin and also contains Tween® and TNBP, as well as residual factor VIII.

### 2) Chromatographic separation

The previously-eluted fraction enriched with von Willebrand factor, constituting one batch of starting fraction containing the von Willebrand factor according to the invention, is loaded into a DEAE-Fractogel®-TSK 650 (M) type chromatography column with a length of 25 cm and a diameter of 1 cm, equilibrated beforehand with the same buffer as that in 1), the osmolarity of which is 387 mosmolkg⁻¹, the linear velocity of which is set at 100 cm/hour. 140 ml of this fraction containing 12.9 IU vWF/ml and 6.6 IU factor VIII/ml, or an R ratio of 51.1% (R= FVIII:C/FvW:RCo) is injected.

The column is then washed with an acidic buffer of 20 mM sodium acetate, adjusted to a pH of 4.35 and 80 mosmolkg⁻¹, with a linear velocity of 150 cm/hour. Under these conditions, a very good elimination not only of the viral inactivation agent residues but also of the fibronectin and above all of factor VIII which was again complexed to the von Willebrand factor is ensured, without precipitation of these proteins being observed in the column. When the O.D. has returned to the baseline, the linear velocity is brought back to 100 cm/hour then the column is rinsed and equilibrated with the same buffer as previously, containing NaCl at 0.11 M.

The fraction containing the von Willebrand factor is eluted by increasing the concentration of the NaCl of the equilibrating buffer to 0.17 M, adjusting to a pH of 6.95 and 492 mosmolkg⁻¹.

The eluted von Willebrand factor fraction then undergoes standard sterilizing filtration treatments on filters of 0.22 µm, nanofiltration on filters of 35 nm, diafiltration and ultrafiltration according to known techniques so that the von Willebrand factor concentrate has a specific activity (S.A.) of at least 90 IU RCo/mg of protein.

Albumin at 10 g/l is added to the von Willebrand factor concentrates thus obtained then it is lyophilized at -40°C for 48 hours. The lyophilization is followed by a heat viral inactivation treatment by heating the lyophilizate to dryness at 80°C for 72 hours.

The process of example 1 allows to obtain a concentrate of von Willebrand factor wherein the residual content of factor VIII is less than or equal to 10IU/100IU vWF/RCo, i.e. less than or equal to 10 IU/ml.

### Example 2: Effect of the composition of the invention on endothelial proliferation

Angiogenesis is a complex process driven by endothelial cells involving multiple steps; crucial amongst these is endothelial proliferation. In this test, we compare the effectiveness of in house purified vWF, from Haemate P® (160 UI/mL of von Willebrand factor and 66,6UI/mL of factor VIII, product of CSL Limited) and the pharmaceutical composition of the invention on the proliferation in vWF-deficient cells.

siCTL- or sivWF-treated HUVECs were incubated with M199 medium containing 1% fetal calf serum (FCS) for 4 hours. Cells were seeded onto gelatin-coated 24-well plates at 3684 cells/cm2 in M199/10% FCS ±100 ng/mL VEGF₁₆₅ (PeproTech) and ± 10 µg/mL in house purified vWF, from Haemate P®. Cells were counted using a hemocytometer 96 hours after seeding.

In house purified vWF, from Haemate P® was used to compare proliferation of control and vWF-deficient endothelial cells seeded on gelatin (as control matrix) or on vWF. Proliferation of control HUVEC was lower on vWF compared to gelatin. Moreover, the enhanced proliferation observed in vWF-deficient HUVEC was significantly reduced by extracellular vWF (Figure 1). Thus in house purified vWF, from Haemate P® does correct the increased proliferation of vWF-deficient endothelial cells.

To determine whether in house purified vWF, from Haemate P® added in solution is also able to rescue the pro-proliferative effect of vWF deficiency, proliferation assays were performed using control or vWFF-deficient EC seeded on gelatin with in house purified vWF, from Haemate P® added in solution to the cells. Addition of in house purified vWF, from Haemate P® in solution to the cell culture media was unable to rescue the pro-proliferative phenotype (Figure 2). These results suggest that the exposure of binding sites on vWF, which occurs upon adhesion to surfaces, may be required for its effects on proliferation.

The effect of the pharmaceutical composition of the invention was then tested in proliferation assay similar to that described in Figure 1. At the same concentration as previously used with plasma-derived in house purified vWF, from Haemate P® (10 µg/mL), the pharmaceutical composition of the invention induced a significant inhibition of proliferation in VWF-deficient cells (Figure 3). The effect was more marked than that observed with in house purified vWF, from Haemate P® ((at 30 ug/ml, see Figure 2). These data indicate that extracellular vWF can compensate for the loss of cellular vWF in this assay, and that the pharmaceutical composition of the invention is particularly efficient in this process. However, in-house purified vWF from Haemate P® was more effective than the pharmaceutical composition of the invention at reducing proliferation of control HUVEC (Figure 3, white bars).

### Example 3: Regulation of angiogenesis by the composition of the invention: molecular pathways studies

### 1) Effect on αvβ3 integrin levels

β3 integrin is one of the two chains that forms the adhesion molecule αvβ3, which plays a crucial role in angiogenesis, partly through regulation of VEGFR2 signalling.

### Measurement of β3 integrin internalization

Figure 5 (parts A, B and C) shows that vWF stabilizes the expression of β3 integrin on the EC surface, since vWF-deficient cells showed decreased expression and increased internalization of β3 from the cell surface. Figures (parts A, B and C) have been published in the publication of Starcke et al., Blood, January 2011, vol 117, n°3, pages 1071 - 1080, see in particular page 1075 for Figure 3.

### Adhesion assay

Plates (96-well) were coated with 1% gelatin, 100 µg/mL type 1 collagen (BD Biosciences), 30 µg/mL vWF (in house purified vWF, from Haemate P® or the composition of the invention), or 1% BSA overnight at 4°C, washed 2 times with PBS, and blocked with 1% BSA for 1 hour. siCTL- or sivWF-treated HUVECs were labeled with 6.25µM 5-chloromethylfluorescein diacetate (Invitrogen) following the manufacturer's protocol, and reseeded at 20 000 cells per well onto the coated plates. After 40 minutes, the total cellular fluorescence was measured with a Synergy plate reader (BioTek Instruments) with absorption and emission settings at 492 and 517 nm, respectively. Wells were then washed gently 3 times with PBS containing Ca²⁺ and Mg²⁺ at 37°C to remove nonadherent cells. The fluorescence of bound cells was then measured with the Synergy plate reader using the same settings. The percentage of bound cells relative to the total number seeded was then calculated.

Adhesion assays to extracellular matrix proteins confirmed the functional defect of αvβ3 integrin in vWF-deficient HUVECs, because binding of ECs to the αvβ3-dependent substrate gelatin was decreased compared with controls (figure 5C). ECs binding to vWF, which is also αvβ3 dependent, was very low and, although lower in vWF-depleted cells, it did not achieve significance. Binding to the β1 integrin ligand collagen type I was unaffected.

The inventors hypothesized that loss of vWF causes destabilization of β3 integrin at the cell surface and that seeding vWF-deficient cells on vWF could rescue this effect.

Figure 6 shows that the composition of the invention is able to significantly normalise integrin β3 levels in vWF-deficient cells (A); in house purified vWF, from Haemate P® (B) seems to be less effective.

### 2) Effect on ANg-2 levels

Another crucial mechanism through which vWF may control angiogenesis is via the growth factor angiopoietin-2 (Ang-2). Loss of vWF resulted in increased Ang-2 levels in the supernatant of the cells. Ang-2 destabilizes blood vessels and acts synergistically with VEGF-A to promote angiogenesis; overexpression of Ang-2 in vivo results in unstable, dysplastic vessels, and a link between increased Ang-2 and angiodysplasia has been shown in human tissues.

In addition, both the composition of the invention and in house purified vWF, from Haemate P® were able to reduce the increase in Ang-2 expression (Figures 7, parts A and B), suggesting that binding of extracellular vWF to EC is able to control Ang-2 synthesis.

To investigate whether the composition of the invention was able to rescue Ang-2 levels, FAK phosphorylation was measured in control and vWF deficient cells after reseeding onto the composition of the invention. Results suggest that the composition of the invention is able to normalise the decrease signalling observed in vWF deficient cells (Figure 8).

### CONCLUSIONS:

In summary, these results show that the effect of vWF deficiency on angiogenic assays in vitro can be corrected, at least in part, with externally added vWF. The composition of the invention appears to have a more potent effect at correcting the pro-proliferative phenotype of vWF-deficiency than in house purified vWF, from Haemate P®.

Only the composition of the invention was able to stabilize β3 integrin on vWF-deficient endothelial cells.

## Claims

1. Pharmaceutical composition comprising von Willebrand factor for use in the prophylaxis and/or the treatment of a pathology mediated by angiogenesis, wherein the content of factor VIII in the pharmaceutical composition is lower than or equal to 30 IU/ml.

2. Pharmaceutical composition comprising von Willebrand factor according to claim 1, wherein the content of factor VIII in the pharmaceutical composition is lower than or equal to 10 IU/ml.

3. Pharmaceutical composition comprising von Willebrand factor according to any one of claims 1 to 2, wherein the pharmaceutical composition is free of ADAMTS13.

4. Pharmaceutical composition comprising von Willebrand factor according to any one of claims 1 to 3, wherein the high-molecular weight multimers represent at least 65%, preferably 70%, preferably 75%, preferably 80% of the total multimer content of von Willebrand factor contained in the pharmaceutical composition.

5. Pharmaceutical composition comprising von Willebrand factor according to any one of claims 1 to 4, for use in the prophylaxis and/or the treatment of a cancer pathology mediated by angiogenesis.

6. Pharmaceutical composition comprising von Willebrand factor according to claim 5, wherein the cancer pathology mediated by angiogenesis is a solid tumour, a blood-borne tumour, a benign tumour or a tumour metastasis.

7. Pharmaceutical composition comprising von Willebrand factor according to any one of claims 5 to 6, wherein the cancer pathology mediated by angiogenesis is breast cancer, leukaemia, Kaposi's sarcoma, hemangioma, rhabdomyosarcoma, retinoblastoma, Ewing's sarcoma, neuroblastoma, osteosarcoma, acoustic neuroma, neurofibroma, trachoma, pyogenic granulomas or other neoplastic diseases of the bone marrow.

8. Pharmaceutical composition comprising von Willebrand factor according to claim 1, wherein the pathology mediated by angiogenesis is selected among the group consisting of : macular degeneration, macular degeneration of age, trachoma, diabetic retinopathy, neovascular glaucoma, retrolental fibroplasia of, proliferative vitreoretinopathy, psoriasis, Kaposi's syndrome, ulcerative colitis, retinopathy of prematurity, of epidemic keratoconjunctivitis, atopic keratitis, the superior limbic keratitis, dry keratoconjunctivitis (pterygium keratitis sicca) of vitamin A deficiency, corneal graft rejection, of A condition resulting from excessive contact lens wear, Sjögrens syndrome, acne rosacea, couperose, phlyctenular keratitis, syphilis, lipid degeneration, infections with Herpes simplex virus, infections by Herpes zoster viruses, protozoal infections, infections of mycobacteria, infections causing a retinitis or choroiditis, chemical burns, bacterial ulcers, fungal ulcers, Mooren's ulcer, of the Terrien's marginal degeneration, marginal keratolysis of from polyarteritis, scleritis, Stevens-Johnson of the disease, radial keratotomy, trauma, systemic lupus, Wegener sarcoidosis, sarcoidosis, of sickle erythrocytes, the vein occlusion, artery occlusion, sickle cell anemia, pseudoxanthoma elasticum, pemphigoid disease, Paget's disease, obstruction of the carotid artery disease, the Chronic vitreous disease, Lyme disease, Eales's disease, Behcet's disease, presumed ocular histoplasmosis, Best's disease, myopia, optic bites, Stargardt disease, disease pars planitis, chronic retinal detachment, optic pits, hyperviscosity syndromes, the taxoplasmose, post-laser complications, abnormal proliferation of fibrovascular or fibrous tissue, bartonellosis , Osler-Weber-Rendu disease, acquired immunodeficiency syndrome, ocular neovascular disease, osteoarthritis, diseases from chronic inflammation, Crohn's disease, atherosclerosis, pyrogenic granulomas and rubella.
